# EUROPEAN PATENT APPLICATION

(11) **EP 2 848 186 A1**
(43) Date of publication of application: **18.03.2015**
(21) Application number: 13787924.3
(22) Date of filing: 25.04.2013
(51) Int. Cl.: A61B 1/00, G02B 23/26

(54) **ENDOSCOPE ILLUMINATION OPTICAL ASSEMBLY AND ENDOSCOPE**

(30) Priority: 11.05.2012 JP 2012109540
(71) Applicant: Olympus Corporation, Shibuya-ku Tokyo 151-0072 (JP)
(72) Inventor: KIKUCHI, Satoru, Tokyo 151-0072 (JP); KOBAYASHI, Hiroyoshi, Tokyo151-0072 (JP)
(74) Representative: Schicker, Silvia
(86) International application number: PCT/JP2013/062285
(87) International publication number: WO 2013/168594

(57) **Abstract**

A wide range is illuminated with a sufficient light amount while a diameter of an insertion portion is reduced. Provided is an illumination optical system for endoscope (1) including an optical fiber (6) for guiding excitation light emitted from a light source; a first mirror (9) and a second mirror (10) disposed facing a light emission end (6a) at a distal end of the optical fiber (6) and diffusing the excitation light emitted from the light emission end (6a); and a phosphor (11) on which the excitation light diffused by the first mirror (9) and the second mirror (10) is incident and which emits fluorescence.

## Description

### {Technical Field}

The present invention relates to an illumination optical system for endoscope and an endoscope.

### {Background Art}

Conventionally, an illumination optical system for endoscope is known which guides light emitted from a xenon lamp or a halogen lamp to a distal end of an insertion portion of an endoscope with use of a light guide formed of a fiber bundle (see PTL 1, for example).

### {Citation List}

### {Patent Literature}

{PTL 1}
Japanese Unexamined Patent Application, Publication No. 2004-16410

### {Summary of Invention}

### {Technical Problem}

In the illumination optical system of PTL 1, however, in order to illuminate a wide range of an object with a sufficient light amount, an optical fiber with large numerical aperture needs to be used, and furthermore a large number of the optical fibers need to be used. Accordingly, most of a cross section of an insertion portion of an endoscope is occupied by the optical fibers, and thus it is difficult for the diameter of the insertion portion to be reduced.

The present invention has been made in view of the aforementioned circumstance, and an object thereof is to provide an illumination optical system for endoscope and an endoscope that can illuminate a wide range with a sufficient light amount while a diameter of an insertion portion is reduced.

### {Solution to Problem}

In order to achieve the aforementioned object, the present invention provides the following solutions.

An aspect of the present invention provides an illumination optical system for endoscope including a light guide member for guiding excitation light emitted from a light source; a light diffusing member which is disposed facing a light emission end at a distal end of the light guide member and diffuses the excitation light emitted from the light emission end; and a phosphor on which the excitation light diffused by the light diffusing member is incident, the phosphor emitting fluorescence.

According to the present aspect, the excitation light emitted from the light source is guided by the light guide member, the excitation light is emitted from the light emission end at the distal end of the light guide member, and then the emitted excitation light is diffused by the light diffusing member, and the diffused excitation light is subsequently incident on the phosphor. On the phosphor, the excitation light is incident, thereby exciting fluorescent substances to generate fluorescence, and the fluorescence is emitted toward an object. In this case, since the excitation light is diffused by the light diffusing member, the illuminated range is made wider than a range that can be obtained depending on numerical aperture of the light guide member. Thereby, the fluorescence can be emitted from the light emission surface that is formed in the wide range. Specifically, a light guide area of the light guide member can be reduced sufficiently, and a diameter of an insertion portion of the endoscope can be reduced.

In the above aspect, a minute optical system may be disposed on the light emission surface of the fluorescence emitted from the phosphor.

In this case, the minute optical system may be a microlens array or a Fresnel lens.

With such a configuration, while a dimension in an optical axis direction is reduced, the emitting direction of the fluorescence, which is emitted in every direction from the phosphor included in a wide range, can be controlled.

In the above aspect, the light diffusing member may include a conical mirror which is disposed at a position to shield the excitation light emitted from the light emission end of the light guide member.

With such a configuration, the excitation light emitted from the light emission end of the light guide member is reflected by the conical mirror so that a conical surface of the mirror causes annular diffusion of the excitation light. Specifically, efficient diffusion of the excitation light with a simple configuration allows the excitation light to enter the phosphor included in a wide range.

In the above aspect, the light diffusing member may include a first mirror which is disposed at a position to shield the excitation light emitted from the light emission end of the light guide member and a second mirror which reflects the excitation light reflected by the first mirror, and at least one of the first mirror and the second mirror may have a curvature that causes diffusion of the excitation light.

With such a configuration, the excitation light emitted from the light emission end of the light guide member is diffused by the mirror which has a curvature that causes diffusion during the reflection performed two times, and thus the fluorescence can be emitted from the phosphor included in a wide range.

Furthermore, another aspect of the present invention provides an endoscope including a plurality of the illumination optical systems for endoscope having any one of the above characteristics, and further including a light receiving unit which receives reflection light, from an object, of the fluorescence emitted from the illumination optical system for endoscope; and a channel including an opening which allows a treatment tool to emerge and retract, wherein the light emission surface of the fluorescence emitted from the illumination optical system for endoscope is respectively disposed at a same side as the light receiving unit and a different side from the light receiving unit with respect to the opening of the channel.

According to the present aspect, the fluorescence is emitted from both sides interposing the channel toward the object. Accordingly, even when the treatment tool is made to protrude from the opening, a shadow of the treatment tool can be prevented from being formed on the object. For this reason, the visibility of the object can be improved.

### {Advantageous Effects of Invention}

The present invention can provide the effect that a wide range can be illuminated with a sufficient light amount while a diameter of an insertion portion is reduced.

### {Brief Description of Drawings}

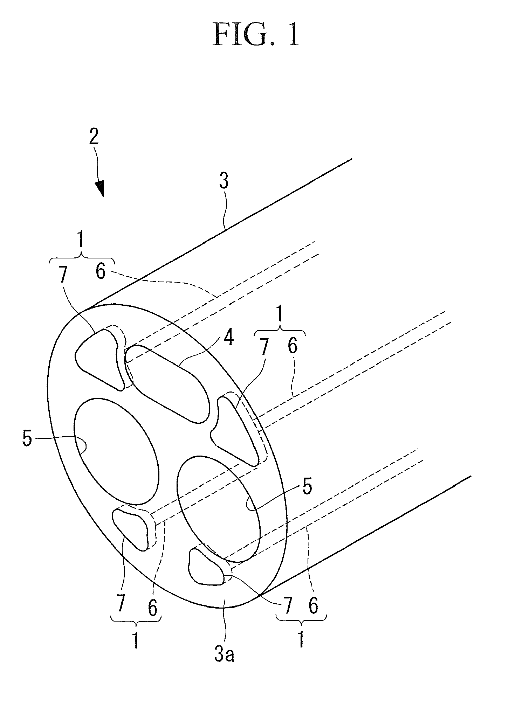

{Fig. 1}
   Fig. 1 is a perspective view for illustrating a distal end portion of an endoscope provided with an illumination optical system for endoscope according to an embodiment of the present invention.
{Fig. 2}
   Fig. 2 is a longitudinal sectional view for illustrating the illumination optical system for endoscope of Fig. 1.
{Fig. 3}
   Fig. 3 is a longitudinal sectional view for illustrating a modification of the illumination optical system for endoscope of Fig. 2.

### {Description of Embodiments}

Hereinafter, a description will be given of an illumination optical system for endoscope 1 and an endoscope 2 according to an embodiment of the present invention with reference to the drawings.

The endoscope 2 of the present embodiment includes, at a distal end of an insertion portion 3, the illumination optical system (illumination optical system for endoscope) 1 of the present embodiment which emits illumination light, an objective lens 4 for collecting reflection light which is emitted from the illumination optical system 1 and is reflected at an object to return, and a forceps channel 5 which is provided across an entire length in the longitudinal direction of the insertion portion 3 and is opened to a distal end surface 3a, as illustrated in Fig. 1.

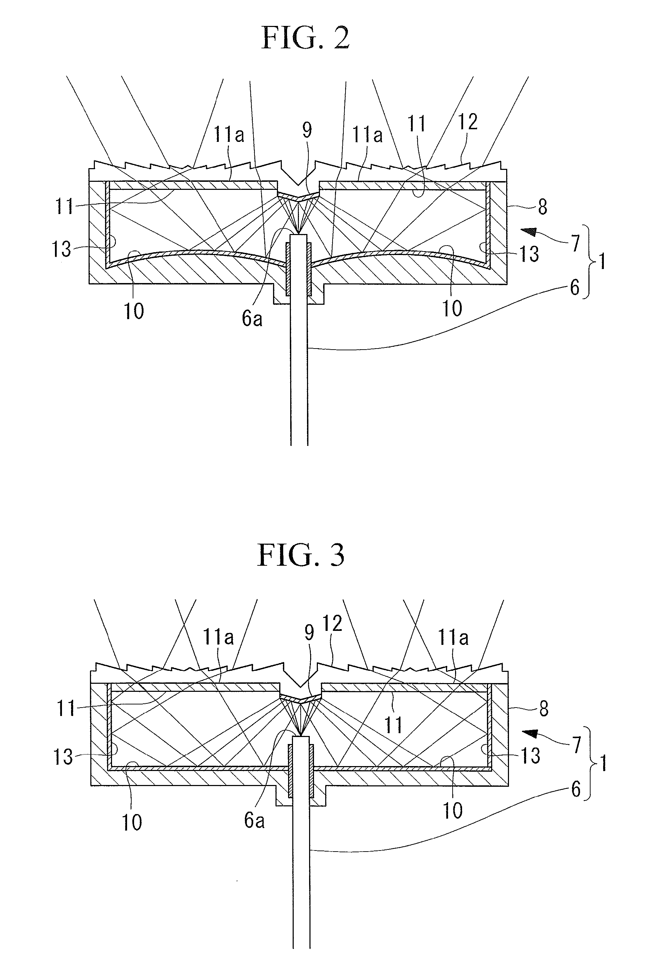

The illumination optical system 1 of the present embodiment includes a plurality of optical fibers (light guide members) 6 which guide excitation light emitted from a light source (not illustrated), a fluorescence generating unit 7 which is attached to an light emission end 6a of each optical fiber 6, as illustrated in Fig. 2.

Each fluorescence generating unit 7 is provided with one optical fiber 6, for example.

In the endoscope 2 of the present embodiment, the illumination optical system 1 is disposed at each of both sides of the objective lens 4 to illuminate the visual field range of the objective lens 4 brightly. At the same time, the illumination optical system 1 is also disposed on a side opposite to the objective lens 4 with the forceps channel 5 therebetween.

The fluorescence generating unit 7 includes a box-shaped case 8, a first mirror 9 disposed in the case 8, a second mirror 10 and a phosphor 11, as illustrated in Fig. 2. The first mirror 9 is disposed in a range illuminated by excitation light that is emitted from the light emission end 6a of the optical fiber 6 so that the first mirror 9 reflects the excitation light.

The second mirror 10 reflects again the excitation light that has been reflected by the first mirror 9. On the phosphor 11, the excitation light reflected by the second mirror 10 is incident so that the phosphor 11 emits fluorescence.

One end surface of the case 8 is formed of a Fresnel lens 12. The phosphor 11 includes a light emission surface 11a which is disposed on a rear side of the Fresnel lens 12 and emits the generated fluorescence toward the Fresnel lens 12.

The first mirror 9 is a conical mirror which is disposed at a position to shield at least a part of the range illuminated by the excitation light emitted from the light emission end 6a of the optical fiber 6. The first mirror 9 is formed of a conical mirror. Thereby, the excitation light is diffused by a convex conical surface of the conical mirror.

The second mirror 10 is disposed at a position facing the first mirror 9 and reflects again the excitation light which has been reflected by the first mirror 9. The second mirror 10 has a convex reflecting surface. As the result, the excitation light reflected by the second mirror 10 is further diffused by the convex reflecting surface.

The phosphor 11 contains fluorescent particles constituted of fluorescent substances in a transparent resin material. The fluorescent substances are excited due to incidence of excitation light, thereby causing fluorescence to be generated in every direction.

Even an inner circumferential surface of the case 8 is coated with a reflecting film 13, and a part of the excitation light reflected by the second mirror 10, which does not directly enter the phosphor 11 but enters the inner circumferential surface of the case 8, is reflected by the reflecting film 13 to be also directed to the phosphor 11 side.

The Fresnel lens 12 disperses the emitting directions of the fluorescence which is generated at the phosphor 11 and is emitted from the light emission surface 11a of the phosphor 11 toward various directions, and adjusts the fluorescence so that an object is irradiated by the fluorescence with a uniform density.

Hereinafter, a description will be given of operation of the illumination optical system 1 and the endoscope 2 of the present embodiment thus formed.

According to the illumination optical system 1 of the present embodiment, the excitation light guided by the optical fiber 6 is emitted from the light emission end 6a disposed in the case 8 of the fluorescence generating unit 7 disposed at the distal end of the optical fiber 6, is reflected by the first mirror 9, is sequentially reflected by the second mirror 10, and is then made pass through the phosphor 11 to generate fluorescence.

In this case, the first mirror 9 and the second mirror 10 each have a convex reflecting surface, and thus the excitation light is diffused every time the light is reflected by the reflecting surface of the respective mirrors 9 and 10, thereby causing expansion of an area illuminated by the excitation light. The excitation light which is widened on the expanded illumination area enters the phosphor 11 disposed on the one end surface of the case 8, and thus an area of the light emission surface 11a from which fluorescence is emitted can be made wide.

The fluorescence that has been emitted from the light emission surface 11a of the phosphor 11 is made pass through the Fresnel lens 12 to be dispersed with a uniform fluorescent density, and then the object is irradiated with the fluorescence.

Specifically, according to the illumination optical system 1 of the present embodiment, the fluorescence to be dispersed over the wide area is generated by the fluorescence generating unit 7, from the excitation light guided by a single or a small number of optical fibers 6. Therefore, the ratio of an area of a cross section of an insertion portion 2 occupied by the optical fibers 6 can be decreased. As the result, the outer diameter of the insertion portion 2 can be made small advantageously.

Moreover, the shape of the phosphor 11, on which the excitation light diffused by the first mirror 9 and the second mirror 10 is incident, can be set relatively freely, thereby enabling an essential configuration in the distal end surface 3a of the insertion portion 3 of the endoscope 2, e.g., disposition of the distal end surface 3a so as to occupy an area excluding the objective lens 4, the forceps channel 5 and the like, as illustrated in Fig. 1. As the result, fluorescence can be emitted from a wide area of the distal end surface 3a of the insertion portion 3, and thus bright illuminating can be achieved advantageously.

Furthermore, according to the illumination optical system 1 of the present embodiment, the excitation light is dispersed while the excitation light is reflected by the first mirror 9 and the second mirror 10. Accordingly, the fluorescence generating unit 7 to be disposed on the distal end surface 3a of the insertion portion 3 can be disposed in an extremely-thin range in the vicinity of the distal end surface 3a, offering an advantage of space saving.

Moreover, according to the endoscope 2 of the present embodiment, the fluorescence generating unit 7 of the illumination optical system 1 is disposed on each of the both sides interposing the forceps channel 5. Accordingly, even at the time of treatment when a treatment tool such as a forceps is made to protrude from the forceps channel 5, there is an advantage that an object can be brightly illuminated without a shadow of the treatment tool being formed on the object. As the result, the visibility of the object can be improved.

In the present embodiment, a mirror that has a convex reflecting surface is adopted as both the first mirror 9 and the second mirror 10. Alternatively, however, the second mirror 10 may be formed of a planar mirror, as illustrated in Fig. 3. Also with such a configuration, excitation light can be diffused due to light-diffusing effect of a conical mirror, i.e., the first mirror 9, and thus illumination light that illuminates a wide range can be obtained.

Alternatively, only the second mirror 10 may have a convex reflecting surface and the first mirror 9 may be formed of a planar mirror. Furthermore, if any one of the first mirror 9 and the second mirror 10 can provide sufficient excitation light-diffusing effect, the other mirror may be formed of a mirror that has a concave reflecting surface.

Moreover, the Fresnel lens 12 is disposed at a position to cover the light emission surface 11a of the phosphor 11. Alternatively, however, a similar minute optical system, e.g., a microlens array (not illustrated) including semi-spherical microlenses arranged therein may be disposed.

### {Reference Signs List}

- 1: illumination optical system (illumination optical system for endoscope)
- 2: endoscope
- 4: objective lens (light receiving unit)
- 5: forceps channel (channel)
- 6: optical fiber (light guide member)
- 6a: light emission end
- 9: first mirror (light diffusing member)
- 10: second mirror (light diffusing member)
- 11: phosphor
- 11a: light emission surface
- 12: Fresnel lens (minute optical system)

## Claims

1. An illumination optical system for endoscope comprising:
a light guide member for guiding excitation light emitted from a light source;
a light diffusing member which is disposed facing a light emission end at a distal end of the light guide member and diffuses the excitation light emitted from the light emission end; and
a phosphor on which the excitation light diffused by the light diffusing member is incident, the phosphor emitting fluorescence.

2. The illumination optical system for endoscope according to claim 1, wherein a minute optical system is disposed on a light emission surface of the fluorescence emitted from the phosphor.

3. The illumination optical system for endoscope according to claim 1 or 2, wherein the light diffusing member includes a conical mirror which is disposed at a position to shield the excitation light emitted from the light emission end of the light guide member.

4. The illumination optical system for endoscope according to any one of claims 1 to 3, wherein the light diffusing member includes a first mirror which is disposed at a position to shield the excitation light emitted from the light emission end of the light guide member and a second mirror which reflects the excitation light reflected by the first mirror, and at least one of the first mirror and the second mirror has a curvature that causes diffusion of the excitation light.

5. The illumination optical system for endoscope according to claim 2, wherein the minute optical system is a microlens array.

6. The illumination optical system for endoscope according to claim 2, wherein the minute optical system is a Fresnel lens.

7. An endoscope comprising a plurality of the illumination optical systems for endoscope according to any one of claims 1 to 6, further comprising:
a light receiving unit which receives reflection light, from an object, of the fluorescence emitted from the illumination optical system for endoscope; and
a channel including an opening which allows a treatment tool to emerge and retract,
wherein light emission surfaces of the fluorescence emitted from the illumination optical system for endoscope are respectively disposed at a same side as the light receiving unit and a different side from the light receiving unit with respect to the opening of the channel.
